(19) **Europäisches Patentamt**
**European Patent Office**
**Office européen des brevets**

(11) **EP 3 199 617 B1**

(12) **EUROPEAN PATENT SPECIFICATION**

(45) Date of publication and mention
of the grant of the patent:
**25.12.2019 Bulletin 2019/52**

(21) Application number: **17154186.5**

(22) Date of filing: **01.02.2017**

(51) Int Cl.:
*C12M 1/12* *(2006.01)*     *B01D 63/06* *(2006.01)*
*B01D 63/16* *(2006.01)*     *C12M 1/26* *(2006.01)*
*C12M 1/00* *(2006.01)*

(54) **SYSTEMS AND METHODS FOR THE SEPARATION OF CELLS FROM MICROCARRIERS USING A SPINNING MEMBRANE**

SYSTEME UND VERFAHREN ZUR TRENNUNG VON ZELLEN VON MIKROTRÄGERN MIT EINER ROTIERENDEN MEMBRAN

SYSTÈMES ET PROCÉDÉS DE SÉPARATION DE CELLULES À PARTIR DES MICROSUPPORTS À L'AIDE D'UNE MEMBRANE ROTATIVE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.02.2016   US 201662289677 P**
**31.01.2017   US 201715421075**

(43) Date of publication of application:
**02.08.2017   Bulletin 2017/31**

(73) Proprietor: **Fenwal, Inc.**
**Lake Zurich, IL 60047 (US)**

(72) Inventors:
• **Wegener, Christopher**
**Lake Zurich, IL 60047 (US)**
• **Min, Kyungyoon**
**Lake Zurich, IL 60047 (US)**

(74) Representative: **Maikowski & Ninnemann**
**Patentanwälte Partnerschaft mbB**
**Postfach 15 09 20**
**10671 Berlin (DE)**

(56) References cited:
**US-A- 5 002 890     US-A- 5 155 034**
**US-A- 5 654 197     US-A1- 2014 199 680**

## Description

Field of the Disclosure

The present disclosure is directed to the methods and systems for processing a suspension of biological cells and microcarriers to separate the cells from the microcarriers. More particularly, the present disclosure is directed to methods and systems for processing the suspension and achieving the separation utilizing a separation device that includes a spinning membrane.

Background

Cell therapies represent an emerging field of medical research. A cell therapy procedure often involves the manipulation of autologous or allogeneic cells for a particular patient or indication. Often, one of these manipulation steps requires in vitro culture/expansion of adherent cell lines. Traditionally, these adherent cells have been grown in culture media in 2D culture vessels, such as T-Flasks or Petri Dishes.

In many applications, the production of as many cells as possible allows for reduction of cost and/or more efficacious treatments. Traditional 2D culture vessels often do not provide enough surface area to accommodate the desired number of cells. One way of providing additional surface area is to grow adherent cells on the surface of plastic microcarriers, small particles that have an affinity for growing cells thereon. Growing cells in this fashion often dramatically increases the number of cells available for further processing and for cell therapy procedures.

Once grown, these cells must be harvested so that they can be reinfused into a patient. Where cells have been grown on a microcarrier, a cleaving agent is often added (e.g., Trypsin or a synthetic alternative) to the culture to separate the grown cells from the microcarriers and allow for resuspension of the now separated cells. Further isolation of the cells or harvesting can be achieved through simple filtration where the larger diameter microcarriers are captured by the filter and the biological cells pass through the membrane. However, where large volumes of the culture are harvested, "dead-end" filtration, i.e., filtration with a single inlet, single outlet and perpendicular flow across the membrane is often inefficient and expensive. Traditional filtration methods may result in a reduction of filtrate flow due to the accumulation of microcarriers on the filter surface. Thus, traditional filtration often requires filters with large surface areas and high up-stream volumes to accomodate the volume of the retained microcarriers.

US 2014/199680 describes a system for washing and processing biological cells and fluids. The system uses a fluid circuit including a spinning membrane separation device.

Accordingly, it would be desirable to provide methods and systems whereby large volumes of suspensions including biological cells and microcarriers can be separated whereby the separated microcarriers are removed and the biological cells are collected for further processing, without the drawbacks of traditional filtration systems.

Summary

In one aspect, the present disclosure is directed to a method for separating biological cells from microcarriers in a suspension. The method includes introducing a suspension of biological cells and microcarriers into a separation device with a relatively rotatable about a central vertical axis cylindrical housing and an internal member. The cylindrical housing has an interior surface and the internal member has an exterior surface. The surfaces define a gap therebetween, wherein one of the surfaces includes a porous membrane with pores sized to retain the microcarriers while allowing the biological cells to pass through the membrane. The suspension is introduced from an inlet at one end of said gap and said microcarriers are removed through an outlet at the opposite end of said gap. The method further includes withdrawing the separated biological cells from the separation device through a different outlet.

The pore size may be less than or equal to approximately 50 $\mu$m. On the other hand, the pore size may be greater than or equal to approximately 20 $\mu$m.

The method may comprise introducing the suspension of biological cells into the gap. The method may also comprise introducing the suspension of biological cells from a container that is in fluid communication with the gap.

The biological cells may be introduced through an inlet in flow communication with the gap and the microcarriers may be removed through an outlet in flow communication with the gap. The biological cells may be adhered to the surface of the microcarriers during the introducing step.

The method may comprise introducing a cleaving agent into the container prior to introducing the biological cells into the separator.

The microcarriers may comprise polymeric, coated beads. The membrane may be made of a material selected from the group of nylon and polycarbonate.

The method may comprise determining the amount of microcarriers and biological cells that are introduced into the separation device. The method may also comprise determining the Total Packed Volume (TCV) Percentage of the suspension being introduced into the separation device. The Total Packed Volume (TCV) Percentage may be determined by:

$$TCV \% = \frac{\text{Total Volume of Cells} + \text{Total Volume of Microcarriers}}{\text{Total Volume of Suspension}}$$

The method may comprise combining the suspension with a cleaving agent neutralizing solution. The biological cells may be selected from the group of white blood cells, red blood cells and stem cells.

In another aspect, the present disclosure is directed to a system for separating biological cells from microcarriers in a suspension that includes a reusable hardware unit with a separation device drive unit for receiving a separation device. The system further includes a disposable fluid circuit mountable on the reusable hardware unit. The disposable fluid circuit includes a separation device with a relatively rotatable cylindrical housing and an internal member, wherein the cylindrical housing has an interior surface and said internal member has an exterior surface, the surfaces defining a gap therebetween. One of the surfaces includes a porous membrane with pores sized to retain the microcarriers while allowing the biological cells to pass through the membrane. The separation device includes an inlet and at least one outlet in fluid communication with the gap. The system also includes a controller configured and/or programmed to control the operation of the separation device.

The controller may be configured and/or programmed to determine the Total Packed Volume % in the suspension. The controller may also be configured and/or programmed to determine a volume of a suspension of biological cells and microcarriers to be introduced into the separation device.

The porous membrane may have a pore size of approximately $20 \mu m$ - $50 \mu m$. In particular, the porous membrane may have a pore size of approximately $30 \mu m$.

Brief Description of the Drawings

Figure 1 is a schematic view of one embodiment of a disposable fluid circuit useful in the systems and methods described herein;

Figure 2 is an enlarged view of the front panel of the reusable processing apparatus;

Figure 3 is a perspective view, partially broken away, of the separation device of Fig. 1;

Figure 4 is another view of the front panel of a reusable processing and/or cell washing apparatus with a disposable fluid circuit mounted thereon;

Figure 5(a) is a schematic view of a suspension of biological cells and microcarriers undergoing separation inside the separation device;

Figure 5(b) is a schematic view of a suspension of biological cells and microcarriers undergoing cleaving and separation inside the separation device; and

Fig. 6 is a flow chart setting forth steps of the method disclosed herein.

Detailed Description of the Embodiments

A description of cell culturing or the process of growing cells on microcarriers is beyond the scope of the present application. Those skilled in the art will recognize the bioreactor systems used to grow the desired cells. such as, but not limited to, GE Healthcare Xuri, PBS Biotech Bioreactor Systems, or Eppendorf/New Brunswick Bio BLU Systems. When the desired cells have achieved sufficient growth in the bioreactor system, the cell/microcarrier aggregates may be drained into a container for subsequent addition of the cleaving agent. Once the cells have been cleaved from the microcarrier substrates and the now separated microcarriers and cells are collected in a container, the container may be directly connected to the system described herein or to a source container that is part of the system. Alternatively, as described below, a source of grown cells still attached to the microcarriers may be directly connected to the system disclosed herein or to a source container that is integrally connected to the system. The uncleaved cell/carrier aggregates may be combined with a cleaving agent in a container that is integrally connected to the system. After a sufficient period of time to effect cleaving, the now cleaved cells and microcarriers are introduced into the system. This way, both the cleaving and separation steps may be performed by the system herein disclosed.

The methods and systems disclosed herein typically employ a reusable separation apparatus and one or more disposable processing circuits adapted for association with the reusable apparatus. The reusable separation apparatus may be any apparatus that can provide for the automated processing of biological cells. By "automated," it is meant that the apparatus can be pre-programmed to carry out the processing steps of a biological fluid processing method without substantial operator involvement. Of course, even in the automated system of the present disclosure, it will be understood that some operator involvement will be required, including the loading or mounting of the disposable fluid circuits onto the reusable apparatus and entering processing parameters. Additional manual steps may be required as well. However, the reusable apparatus can be programmed to perform the cleaving of cells from the microcarriers and the processing of the biological cells and microcarriers through the disposable circuit(s) described below without substantial operator intervention.

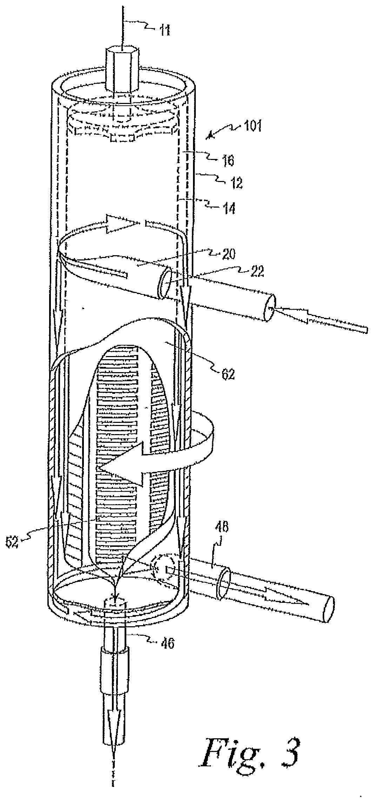

The reusable processing apparatus is capable of effecting the separation of biological cells from particles, such as microcarriers or other synthetic substrates. Thus, the reusable apparatus may generate conditions which allow for the separation of biological cells from such particles. In accordance with the present disclosure, one preferred means for separating biological cells from the particles or substrates is an apparatus that uses a spinning porous membrane to separate one component from other components. An example of such apparatus is the Autopheresis C® sold by Fenwal, Inc. of Lake Zurich, Illinois. A detailed description of a spinning membrane may be found in U.S. Patent No. 5,194,145 to Schoendorfer, and in International (PCT) Application No. PCT/US2012/028492, filed March 9, 2012. In addition, systems and methods that utilize a spinning porous membrane are also disclosed in U.S. Provisional Patent Application No. 61/537,856, filed on September 22, 2011, International (PCT) Application No. PCT/US2012/028522, filed March 9, 2012, International (PCT) Application No. PCT/US2012/054859, filed September 12, 2012 and U.S. Patent Application No. 14/574,539 filed December 18, 2014. The references identified above describe a membrane covered spinner having an interior collection system disposed within a stationary shell. While a detailed discussion of the separation device is beyond the scope of this application, the spinning membrane separation device is shown in Fig. 3 and is briefly discussed below.

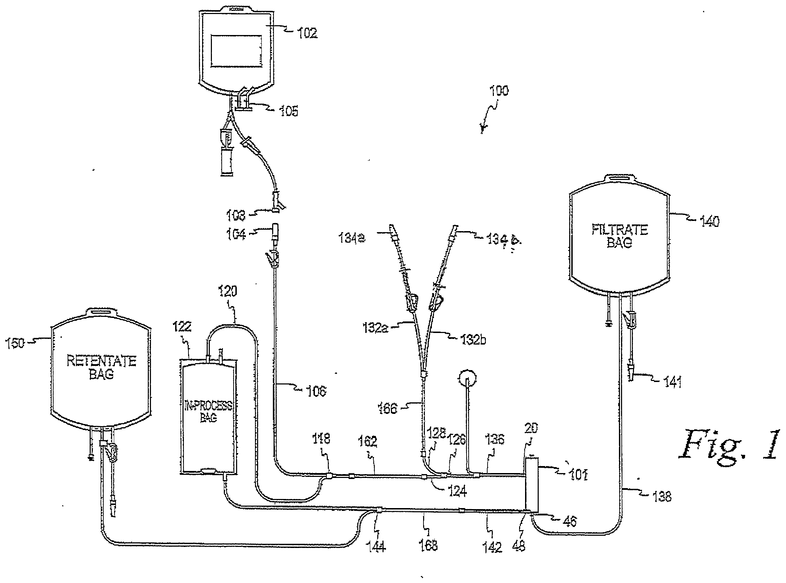

Turning first to Fig. 1, the systems described herein preferably include a disposable fluid circuit for use in the processing and separation of the suspension of biological cells and microcarriers. Fluid circuit 100 is adopted for mounting onto the reusable hardware component, described below. Circuit 100 may include an integrated separation device, such as, but not limited to, the spinning membrane 101 described herein. Circuit 100 may also include filtrate bag or container 140, retentate container or bag 150, and in-process container 122. Disposable fluid circuits of the type described below may further include sampling assemblies (not shown) for collecting samples of biological cells, "final" cell product, or other intermediate products obtained during the biological fluid processing.

As will be seen in the Figures and described in greater detail below, the disposable fluid processing circuits include tubing that defines flow paths throughout the circuit, as well as access sites for sterile or other connection to containers of processing solutions, such as wash solutions, treating (e.g., cleaving) agents, and sources of the biological cell and microparticle suspension fluid. As will be apparent from the disclosure herein, source containers may be attached in sterile fashion to the circuit 100.

As shown in Fig. 1, the tubing of circuit 100 includes spaced tubing segments identified by reference numerals 162, 166, 168. The tubing segments are provided for mating engagement with the peristaltic pumps of the reusable hardware apparatus 200 discussed below. The containers and the plastic tubing are made of conventional medical grade plastic that can be sterilized by sterilization techniques commonly used in the medical field, such as, but not limited to, radiation or autoclaving. Plastic materials useful in the manufacture of containers and tubing in the circuits disclosed herein include plasticized polyvinyl chloride. Other useful materials include acrylics. In addition, certain polyolefins may also be used.

The biological cell/particle (microcarrier) suspension to be processed is typically provided in a source container 102, shown in Fig. 1 as (initially) not connected to the disposable set. As noted above, source container 102 may be attached (in sterile fashion) at the time of use. Source container 102 has one or more access sites 103, 105, one of which may be adapted for (sterile) connection to fluid circuit 100 at docking site 104. Preferably, source containers may be attached in a sterile manner by employing sterile docking devices, such as the BioWelder, available from Sartorius AG, or the SCD IIB Tubing Welder, available from Terumo Medical Corporation. A second access port 105 may also be provided for extracting fluid from the source container 102.

In another embodiment, source container 102 may be pre-attached to circuit 100. In such embodiment, the biological cell/particle suspension may be transferred, in sterile fashion, from a container that is used to grow the cells on the microcarriers.

With further reference to Fig. 1, tubing 106 is connected to downstream branched-connector 118. Branched-connector 118 communicates with tubing 106 and tubing 120, which provides a fluid flow path from "in-process" container 122, described in greater detail below. Tubing segment 124 extends from branched-connector 118 and is joined to a port of further downstream branched-connector 126. A separate flow path defined by tubing 128 is also connected to a port of branched-connector 126.

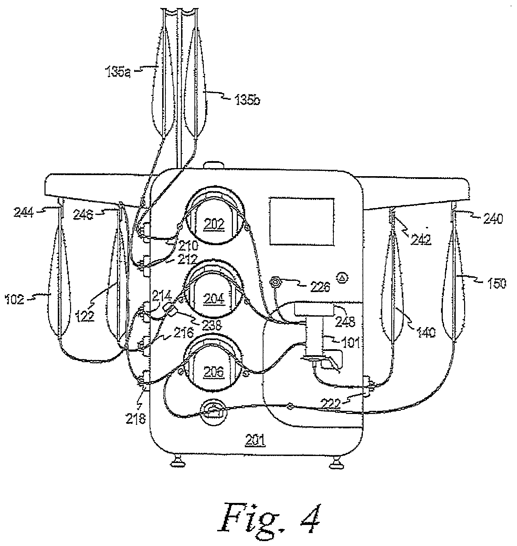

In accordance with the fluid circuit of Fig. 1, a container of wash or other processing/treating solution may be attached (or pre-attached) to set 100. As shown in Fig. 1, tubing 132a (defining a flow path) preferably includes and terminates in an access site such as spike connector 134a. Access site 134a is provided to establish flow communication with a container 135 (shown in Fig. 4) of a wash fluid, such as saline or other solution. More preferably, flow communication between tubing 132a and a container of wash solution may be achieved by sterile connection device, such as, but not limited to, the previously mentioned Terumo SCD IIB. The wash medium or fluid flows from the wash fluid source through tubing segment 132a, and then passes through tubing 128 to the input of the branched-connector 126 described above.

Additional access sites such as site 134b may also be provided. Such additional access sites may be used to establish fluid communication with other solutions and/or agents (e.g., bag 135b shown in Fig.4). For example, in one embodiment, access site 134a or 134b may be used to establish fluid communication with a container including a cleaving agent for separating the biological cells from the surface of the microcarriers. A container of a cleaving agent neutralizing solution may also be connected to circuit 100. In short, it will be understood that solutions such as one or more of a wash solution, a cleaving agent and a cleaving agent neutralizing solution may be attached to fluid circuit at access sites 134a, 134b and additional access sites, as necessary.

As shown in Fig. 1, tubing segment 136 defines a flow path connected at one end to branched-connector 126 and to an inlet port 20 of the separator 101. Preferably, in accordance with the present disclosure, separation device 101 is a spinning membrane separator of the type described in U.S. Patent No. 5,194,145 and U.S. Patent No. 5,053,121, U.S. Provisional Patent Application Serial No. 61/451,903 and PCT/US2012/028522.

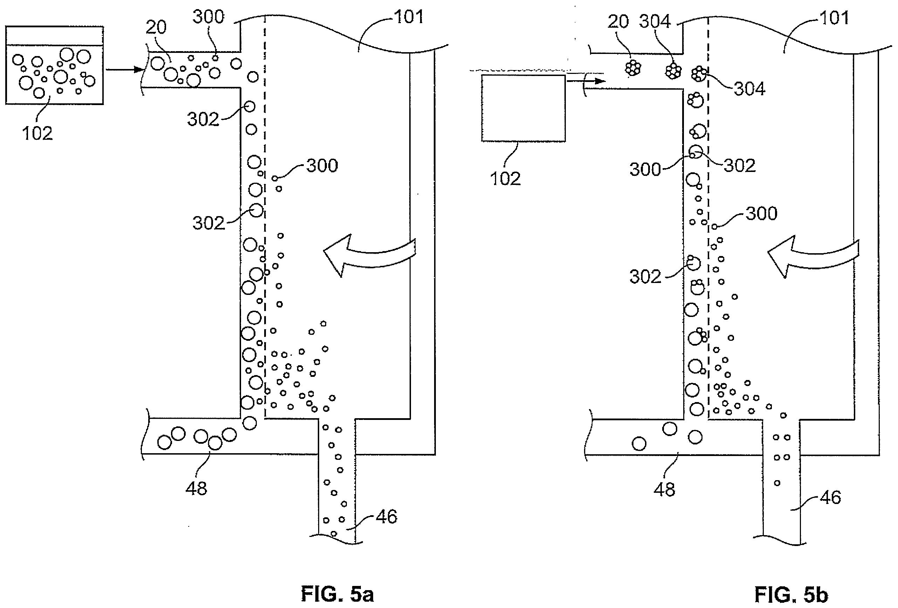

As shown in Fig. 1 (and described in greater detail in connection with Figs. 3, 5(a)-5(b)), the spinning membrane separator 101 has at least two outlet ports. Outlet 46 of separator 101 receives the separated filtrate (e.g., separated biological cells) and is connected to tubing 138, which defines a flow path to filtrate/cell container 140. The filtrate/cell container may further include connection port 141 for sampling the contents within the filtrate/cell container 140.

Separation device 101 preferably includes a second outlet 48 that is connected to tubing segment 142 for directing the microcarriers to branched-connector 144, which branches into and defines a flow path to one or more in-process containers 122 and/or a flow path to a retentate container 150.

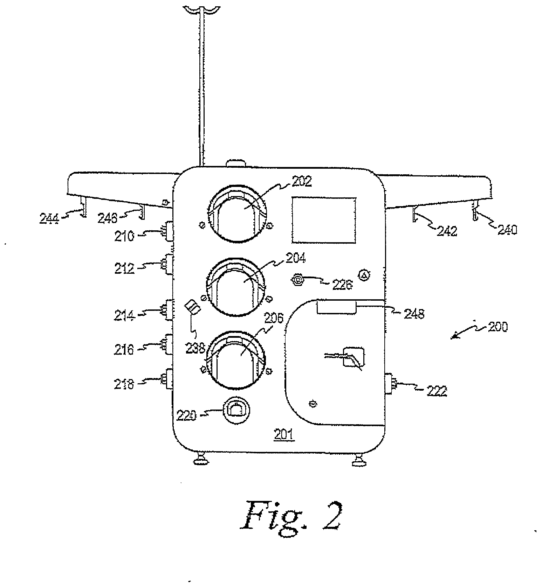

Fig. 2 shows the front panel 201 of reusable hardware processing apparatus 200. Apparatus 200 may be of compact size suitable for placement on a table top of a lab bench and adapted for easy transport. Alternatively, apparatus 200 may be supported by a pedestal that can be wheeled to its desired location. In any event, as shown in Fig. 2, apparatus 200 includes a plurality of peristaltic pumps, such as pumps 202, 204 and 206 on front panel 201. Pump segments of the disposable fluid circuit (described above) are selectively associated with peristaltic pumps 202, 204, and 206. The peristaltic pumps articulate with the fluid sets of Fig. 1 at the pump segments identified by reference numerals 162, 166, 168 and advance the cell suspension or other fluid within the disposable set, as will be understood by those of skill in the art. Apparatus 200 also includes clamps 210, 212, 214, 216, and 218. Clamps 210, 212, 214, 216 and 218 are used to control the flow of the cell suspension through different segments of the disposable set, as described above.

Apparatus 200 also includes several sensors to measure various conditions. The output of the sensors is utilized by device 200 to operate one or more processing or wash cycles. One or more pressure transducer sensor(s) 226 may be provided on apparatus 200 and may be associated with a disposable set 100 at certain points to monitor the pressure during a procedure. Pressure transducer 226 may be integrated into an in-line pressure monitoring site (at, for example, tubing segment 136), to monitor pressure inside separator 101. Air detector sensor 238 may also be associated with the disposable set 100, as necessary. Air detector 238 is optional and may be provided to detect the location of fluid/air interfaces.

Apparatus 200 includes weight scales 240, 242, 244, and 246 from which the cell container, in-process container, source container, and any additional container(s) (e.g., wash solution container, cleaving agent container, retentate container), respectively, may depend and be weighed. The weights of the bags are monitored by weight sensors and recorded during a washing or other procedure. From measurements of the weight sensors, the device, under the direction of the controller, determines whether each container is empty, partially full, or full and controls the components of apparatus 200, such as the peristaltic pumps and clamps 210, 212, 214, 216, 218, 220, 222, and 224.

Apparatus 200 includes at least one drive unit or "spinner" 248 (Fig. 4), which causes the indirect driving of the spinning membrane separator 101. Spinner 248 may consist of a drive motor connected and operated by apparatus 200, coupled to turn an annular magnetic drive member including at least a pair of permanent magnets. As the annular drive member is rotated, magnetic attraction between corresponding magnets within the housing of the spinning membrane separator cause the spinner within the housing of the spinning membrane separator to rotate.

Turning to Fig. 3, a spinning membrane separation device, generally designated 101, is shown. Such a device 101 forms part of the disposable circuit 100. Device 101 includes a generally cylindrical housing 12, mounted concentrically about a longitudinal vertical central axis. An internal member 14 is mounted concentric with the central axis 11. Housing 12 and internal member 14 are relatively rotatable. In the preferred embodiment, as illustrated, housing 12 is stationary and internal member 14 is a rotating spinner that is rotatable concentrically within cylindrical housing 12, as shown by the thick arrow in Fig. 3. The boundaries of the blood flow path are generally defined by gap 16 between the interior surface of housing 12 and the exterior surface of rotary spinner 14. The spacing between the housing and the spinner is sometimes referred to as the shear gap. In one non-limiting example, the shear gap may be approximately 0.025 -0.050 inches (0.067-0.127 cm) and may be of a uniform dimension along axis 11, for example, where the axis of the spinner and housing are coincident. The shear gap may also vary circumferentially for example, where the axis of the housing and spinner are offset.

The shear gap also may vary along the axial direction, for example preferably an increasing gap width in the direction of flow. Such a gap width may range from about 0.025 to about 0.075 inches (0.06 - 0.19 cm). The gap width could be varied by varying the outer diameter of the rotor and/or the inner diameter of the facing housing surface. The gap width could change linearly or stepwise or in some other manner as may be desired. In any event, the width dimension of the gap is preferably selected so that at the desired relative rotational speed, Taylor-Couette flow, such as Taylor vortices, are created in the gap.

In accordance with the present disclosure, the biological cell/ microcarrier suspension is fed from an inlet conduit 20 through an inlet orifice 22, which directs the fluid into the fluid flow entrance region in a path tangential to the circumference about the upper end of the spinner 14. At the bottom end of the cylindrical housing 12, the housing inner wall includes an exit orifice 48.

In the illustrated embodiment, the surface of the rotary spinner 14 is at least partially, and is preferably substantially or entirely, covered by a cylindrical porous membrane 62. The membrane 62 typically has a nominal pore size sufficient to exclude the microcarriers while allowing the biological cells to pass through. In one embodiment, membrane 62 typically has a nominal pore size of approximately 20 $\mu$m - 50 $\mu$m, and more preferably approximately 30 $\mu$m, but other pore sizes may alternatively be used.

Of course, the pore size of membrane will be determined by the diameters of the microcarriers and adherent cells. The pores of membrane 62 should be sized to prevent passage of the microcarriers 302 (Figs. 5a and 5b) but allow the desired cells to pass through the membrane. A typical diameter for microcarriers is 90-220 $\mu$m.

Membranes useful in the methods described herein may be fibrous mesh membranes, cast membranes, track-etched membranes or other types of membranes that will be known to those of skill in the art. For example, in one embodiment, the membrane may be made of a thin (approximately 10-15 $\mu$m thick) sheet of, for example, polycarbonate. In this embodiment, pores (holes) may be cylindrical and larger than those described above. For example, pores may be approximately 20-50 $\mu$m and more preferably about 30 $\mu$m. As noted above, the pores may be sized to allow the desired biological cells (e.g., white blood cells, red blood cells, stem cells) to pass, while the carriers (e.g., microcarriers) are retained within gap 16 for removal from separator 101.

Many of the steps of the method of processing disclosed herein are performed by a software driven microprocessing unit or "controller" of apparatus 200, with certain steps performed by the operator, as noted. For example, the apparatus 200 is switched on, and conducts self-calibration checks, including the checking of the peristaltic pumps, clamps, and sensors. Apparatus 200 under the direction of the controller then prompts the user to enter selected procedural parameters, such as the processing procedure to be performed, the amount of cell suspension to be processed, the number of cycles to take place, etc. The operator may then select and enter the procedural parameters for the separation procedure. The controller may also direct or effect other steps of the method which will now be described.

In accordance with the present disclosure, a method for separating biological cells from microcarriers or other substrate on which the cells have been grown is provided. The method utilizes the disposable fluid circuit 100 of the type described above, mounted on the reusable processing apparatus 200. After the circuit 100 is mounted, a source of biological cells and microcarriers is provided to the circuit. In one embodiment, the source (i.e., suspension) of biological cells and microcarriers may be transferred from a container in which the cells have been grown or in which the cells have been cleaved from the microcarriers. In another embodiment, the biological cells and microcarriers may be provided from a container wherein the cells and microcarriers have been drained after cleaving. In any event, the biological cells and microcarriers may be transferred, in sterile fashion, to source container 102 of circuit 100. Alternatively, source container 102 with the suspension of biological cells and microcarriers already contained therein may be directly attached, likewise in sterile fashion, to circuit 100. Thus, the source of the suspension to be processed will include at least the biological cells and microcarriers. The suspension may further include some of the culture media in which the cells were grown and/or cleaving agent used to effect cleaving of the cells from the microcarrier. Once circuit 100 has been loaded onto apparatus 200, the system will initiate and undergo system checks to ensure proper loading of the circuit 100.

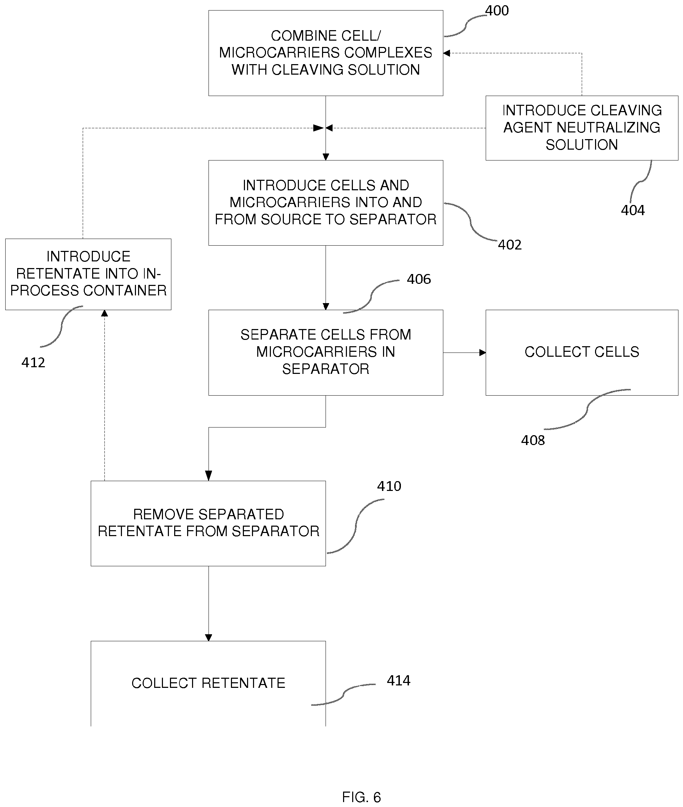

In one embodiment, if not already cleaved, the biological cells are cleaved from the microcarriers prior to introduction into circuit 100 (Fig. 6, step 400). The system may be programed to deliver a selected amount of a cleaving solution from a container (not shown) connected to circuit 100 at one of access sites 134a or 134b. A selected period of time may be provided to allow effective cleaving to take place within container 102. A suitable cleaving solution may be the above-described Trypsin or other synthetic alternative. Prior to introduction of biological cells and microcarriers into separator 101, the system (under the direction of the controller) may initiate a priming of the flow paths of the circuit. The circuit may be primed with a priming solution, such as wash solution, a cleaving solution or other solution such as the cleaving agent neutralizing solution suspended from one more hangers/weight scales of the reusable separation apparatus.

After the system has been primed, the controller directs one or more peristaltic pumps to rotate and draw the suspension from source container 102 through the flow paths of circuit 100. The suspension of biological cells and microcarriers is introduced into separator 101 through inlet 20 where it enters gap 16 (Fig. 6, step 402). If the concentration of biological cells and microcarriers is too high, the suspension of cells and microcarriers may be diluted with saline, additional cleaving agent, the cleaving agent neutralizing solution or other solution. Under the influence of the forces (e.g., Taylor vortices) generated by the spinning action of separator 101, the larger microcarriers are separated from the biological cells. Biological cells 300 (see Fig. 5a) are able to pass through the membrane 62 and into the interior of separator 101 from which they exit via outlet 46 (Fig. 6, step 406). The biological cells flow through flow path 138 and may be collected in cell/filtrate bag 140 for further processing or treatment (Fig. 6, step 408). For example, the now separated and collected cells may provide the source of biological cells that are processed in accordance with the methods and systems described in U.S. Patent Application No. 14/122,855. The remaining microcarriers are too large to pass through membrane 62 and exit gap 16 through outlet 48 together with any unseparated cell/carrier aggregates and any other suspension components that do not pass through membrane 62 (collectively, referred to as "retentate"). Retentate exits gap 16 of separator 101 and flows (under the influence of one or more pumps) to one or both of retentate bag 150 or in-process bag 122 (Fig. 6, step 412). As shown in Fig. 5(b), some of the cells 300 may still be attached to microcarriers 302 when entering gap 16. The spinning (agitating) action of separator 101 during initial introduction into gap 16 or in subsequent cycles of processing of the retentate from in-process container 122 may assist in separating the cells from the microcarriers.

In one embodiment, where the amount of collected cells (as measured by one of weight scales 240, 242, 244 or 246) is determined to be low, the controller will direct flow of the suspension through outlet 48 to in-process container 122. The suspension of microcarriers and residual biological cells may then be withdrawn/pumped from in-process container 122 to separator 101 and, optionally, combined with additional wash solution such as a cleaving agent neutralizing solution, for one or more additional separation cycles, as needed.

As indicated above, a cleaving agent neutralizing solution may be added to the suspension before or during processing (Fig. 6, step 404). Cleaving agent neutralizing agent may be supplied from a container attached in sterile fashion to source container 102 prior to processing. At one of access sites 134a or 135b under the direction of the controller, a selected volume of cleaving agent neutralizing solution may be metered (e.g., pumped) into source container 102 or line 136 where it is combined with the suspension of cells and microcarriers.

As noted above, in accordance with the method of the present disclosure, the suspension of biological cells may be diluted (or further diluted) to avoid introducing too many of the microparticles and cells into gap 16 of separator 101, which may affect (negatively) the efficiency of the separation. Thus, the system may be programmed to determine a Total Cell Volume (TCV) % for the incoming stream of cells and microcarriers. The TCV % may be determined by the following equation:

$$TCV\ \% = \frac{Total\ Volume\ of\ Cells + Total\ Volume\ of\ Microcarriers}{Total\ Volume\ of\ Suspension}$$

A suitable TCV may depend in part on the rotational speed of the spinning membrane 101, the filtrate flux, the size and concentration and the membrane pore size. To arrive at the desired TCV % for a given procedure, the system, under the direction of the controller, delivers the required volume of liquid from the container 135a of wash or other solution suspended from one of weight scales 240, 242, 244 or 246.

**Claims**

1. A method for separating biological cells from microcarriers in a suspension comprising:

   a) introducing a suspension of biological cells and microcarriers into a separation device comprising a relatively rotatable about a central vertical axis cylindrical housing and an internal member, wherein said cylindrical housing has an interior surface and said internal member has an exterior surface, said surfaces defining a gap therebetween, wherein one of said surfaces includes a porous membrane comprising pores sized to retain said microcarriers while allowing said biological cells to pass through said membrane;
   b) introducing said suspension from an inlet at one end of said gap;
   c) removing said microcarriers through an outlet at the opposite end of said gap;
   d) withdrawing said biological cells that have passed through said porous membrane from said separation device through a different outlet.

2. The method of Claim 1, wherein said pore size is less than or equal to approximately 50 $\mu$m and greater than or equal to approximately 20 $\mu$m.

3. The method of any one of Claims 1 through 2 comprising introducing said suspension of biological cells from a container that is in fluid communication with said gap.

4. The method of any one of Claims 1 through 3 wherein said biological cells are adhered to the surface of said microcarriers during said introducing step.

5. The method of Claim 3, further comprising introducing a cleaving agent into said container prior to introducing said biological cells into said separator.

6. The method of any one of Claims 1 through 5, further comprising determining the amount of microcarriers and biological cells that are introduced into said separation device.

7. The method of Claim 6 comprising determining the Total Packed Volume (TCV) Percentage of said suspension being introduced into said separation device.

8. The method of Claim 7, wherein said Total Packed Volume (TCV) Percentage is determined by:

$$TCV\ \% = \frac{\text{Total Volume of Cells} + \text{Total Volume of Microcarriers}}{\text{Total Volume of Suspension}}$$

9. The method of Claim 1, further comprising combining said suspension with a cleaving agent neutralizing solution.

10. The method of any one of Claims 1 through 9, wherein said biological cells are selected from the group of white blood cells, red blood cells and stem cells.

11. A system for separating biological cells from microcarriers in a suspension comprising:

a) a reusable hardware unit comprising a separation device drive unit for receiving a separation device;
b) a disposable fluid circuit mountable on said reusable hardware unit, said disposable fluid circuit including a separation device comprising a relatively rotatable about a central vertical axis cylindrical housing and an internal member, wherein said cylindrical housing has an interior surface and said internal member has an exterior surface, said surfaces defining a gap therebetween, wherein one of said surfaces includes a porous membrane comprising pores sized approximately between 20 $\mu$m-50 $\mu$m to retain said microcarriers while allowing said biological cells to pass through said membrane, said separation device including an inlet at one end of said gap and at least one outlet in fluid communication with said gap said at least one outlet located at an opposite end of said gap; and
c) a controller configured and/or programmed to control the operation of said separation device.

12. The system of Claim 11, wherein said controller is configured and/or programmed to determine the Total Packed Volume % in said suspension.

13. The system of Claim 12, wherein said controller is configured and/or programmed to determine a volume of a suspension of biological cells and microcarriers to be introduced into said separation device.

14. The system of any one of Claims 11 through 13, wherein said porous membrane has a pore size of approximately 30 $\mu$m.

**Patentansprüche**

1. Verfahren zum Trennen von biologischen Zellen von Mikroträgern in einer Suspension, umfassend:

a) Einführen einer Suspension von biologischen Zellen und Mikroträgern in eine Trennvorrichtung, umfassend ein relativ drehbares um eine zentrale vertikale Achse zylindrisches Gehäuse und ein Innenelement, wobei das zylindrische Gehäuse eine innere Oberfläche und das Innenelement eine äußere Oberfläche aufweist, wobei die Oberflächen dazwischen einen Spalt definieren, wobei eine der Oberflächen eine poröse Membran enthält, die Poren umfasst, die so bemessen sind, dass sie die Mikroträger zurückhalten, während die biologischen Zellen die Membran passieren können;
b) Einführen der Suspension aus einem Einlass an einem Ende des Spalts;
c) Entfernen der Mikroträger durch einen Auslass am gegenüberliegenden Ende des Spalts;
d) Entnehmen der biologischen Zellen, die die poröse Membran passiert haben, aus der Trennvorrichtung durch einen anderen Auslass.

2. Verfahren nach Anspruch 1, wobei die Porengröße kleiner oder gleich ungefähr 50 $\mu$m und größer oder gleich ungefähr 20 $\mu$m ist.

3. Verfahren nach einem der Ansprüche 1 bis 2, umfassend das Einführen der Suspension von biologischen Zellen aus einem Behälter, der in Fluidverbindung mit dem Spalt steht.

4. Verfahren nach einem der Ansprüche 1 bis 3, wobei die biologischen Zellen während des Einführschritts an der Oberfläche der Mikroträger angehaftet werden.

5. Verfahren nach Anspruch 3, ferner umfassend das Einführen eines Spaltmittels in den Behälter vor dem Einbringen der biologischen Zellen in den Separator.

6. Verfahren nach einem der Ansprüche 1 bis 5, ferner umfassend das Bestimmen der Menge von Mikroträgern und biologischen Zellen, die in die Trennvorrichtung eingeführt werden.

7. Verfahren nach Anspruch 6, umfassend das Bestimmen des Prozentsatzes des gepackten Gesamtvolumens (TCV) der Suspension, die in die Trennvorrichtung eingeführt wird.

8. Verfahren nach Anspruch 7, wobei der Prozentsatz des gepackten Gesamtvolumens (TCV) bestimmt wird durch:

$$TCV\% = \frac{\text{Gesamtvolumen der Zellen + Gesamtvolumen der Mikroträger}}{\text{Gesamtvolumen der Suspension}}$$

9. Verfahren nach Anspruch 1, ferner umfassend das Kombinieren der Suspension mit einer Spaltmittel-Neutralisationslösung.

10. Verfahren nach einem der Ansprüche 1 bis 9, wobei die biologischen Zellen ausgewählt sind aus der Gruppe aus weißen Blutkörperchen, roten Blutkörperchen und Stammzellen.

11. System zum Trennen von biologischen Zellen von Mikroträgern in einer Suspension, umfassend:

a) eine wiederverwendbare Hardwareeinheit, umfassend eine Trennvorrichtungs-Antriebseinheit zum Aufnehmen einer Trennvorrichtung;
b) einen Einweg-Fluidkreislauf, der an der wiederverwendbaren Hardwareeinheit montierbar ist, wobei der Einweg-Fluidkreislauf eine Trennvorrichtung umfasst, die ein relativ drehbares um eine zentrale vertikale Achse zylindrisches Gehäuse und ein Innenelement umfasst, wobei das zylindrische Gehäuse eine innere Oberfläche und das Innenelement eine äußere Oberfläche aufweist, wobei die Oberflächen dazwischen einen Spalt definieren, wobei eine der Flächen eine poröse Membran enthält, die Poren umfasst, die eine Größe von ungefähr zwischen 20 pm-50 $\mu$m aufweisen, um die Mikroträger zurückzuhalten, während die biologischen Zellen die Membran passieren können, wobei die Trennvorrichtung einen Einlass an einem Ende des Spalts und mindestens einen Auslass in Fluidverbindung mit dem Spalt aufweist, wobei der mindestens eine Auslass an einem gegenüberliegenden Ende des Spalts liegt; und
c) eine Steuerung, die konfiguriert und/oder programmiert ist, um den Betrieb der Trennvorrichtung zu steuern.

12. System nach Anspruch 11, wobei die Steuerung konfiguriert und/oder programmiert ist, um den % des gepackten Gesamtvolumens in der Suspension zu bestimmen.

13. System nach Anspruch 12, wobei die Steuerung konfiguriert und/oder programmiert ist, um ein Volumen einer Suspension von biologischen Zellen und Mikroträgern zu bestimmen, die in die Trennvorrichtung eingeführt werden sollen.

**14.** System nach einem der Ansprüche 11 bis 13, wobei die poröse Membran eine Porengröße von ungefähr 30 μm aufweist.

### Revendications

**1.** Procédé de séparation de cellules biologiques de microsupports dans une suspension comprenant :

a) l'introduction d'une suspension de cellules biologiques et de microsupports dans un dispositif de séparation comprenant un boîtier cylindrique relativement rotatif autour d'un axe central vertical et un élément interne, ledit boîtier cylindrique ayant une surface intérieure et ledit élément interne ayant une surface extérieure, lesdites surfaces définissant un interstice entre elles, une desdites surfaces comportant une membrane poreuse comprenant des pores dimensionnés pour retenir lesdits microsupports tout en laissant passer lesdites cellules biologiques à travers ladite membrane ;
b) l'introduction de ladite suspension depuis une entrée à une extrémité dudit interstice ;
c) le retrait desdits microsupports par une sortie à l'extrémité opposée dudit interstice ;
d) le retrait desdites cellules biologiques qui ont traversé ladite membrane poreuse dudit dispositif de séparation par une sortie différente.

**2.** Procédé de la revendication 1, dans lequel ladite taille de pores est inférieure ou égale à environ 50 μm et supérieure ou égale à environ 20 μm.

**3.** Procédé de l'une quelconque des revendications 1 à 2 comprenant l'introduction de ladite suspension de cellules biologiques à partir d'un récipient qui est en communication fluidique avec ledit interstice.

**4.** Procédé de l'une quelconque des revendications 1 à 3 dans lequel lesdites cellules biologiques sont collées à la surface desdits microsupports pendant ladite étape d'introduction.

**5.** Procédé de la revendication 3, comprenant en outre l'introduction d'un agent du clivage dans ledit récipient avant l'introduction desdites cellules biologiques dans ledit séparateur.

**6.** Procédé de l'une quelconque des revendications 1 à 5, comprenant en outre la détermination de la quantité de microsupports et de cellules biologiques qui sont introduits dans ledit dispositif de séparation.

**7.** Procédé de la revendication 6 comprenant la détermination du pourcentage de volume tassé total (TCV) de ladite suspension introduite dans ledit dispositif de séparation.

**8.** Procédé de la revendication 7, dans lequel ledit pourcentage de volume tassé total (TCV) est déterminé par :

$$\% \ TCV = \frac{\text{Volume total de cellules + Volume total de microsupports}}{\text{Volume total de suspension}}$$

**9.** Procédé de la revendication 1, comprenant en outre la combinaison de ladite suspension avec une solution de neutralisation d'agent de clivage.

**10.** Procédé de l'une quelconque des revendications 1 à 9, dans lequel lesdites cellules biologiques sont choisies dans le groupe constitué par les globules blancs, les globules rouges et les cellules souches.

**11.** Système destiné à séparer des cellules biologiques de microsupports dans une suspension comprenant :

a) une unité matérielle réutilisable comprenant une unité d'entraînement de dispositif d'exploration destinée à recevoir un dispositif de séparation ;
b) un circuit fluidique jetable montable sur ladite unité matérielle réutilisable, ledit circuit fluidique jetable comprenant un dispositif de séparation comprenant un boîtier cylindrique relativement rotatif autour d'un axe central vertical et un élément interne, ledit boîtier cylindrique ayant une surface intérieure et ledit élément interne ayant une surface extérieure, lesdites surfaces définissant un interstice entre elles, une desdites surfaces comportant une membrane poreuse comprenant des pores dimensionnés entre environ 20 $\mu$m et 50 $\mu$m pour retenir lesdits microsupports tout en laissant passer lesdites cellules biologiques à travers ladite membrane, ledit dispositif de séparation comportant une entrée à une extrémité dudit interstice et au moins une sortie en communication fluidique avec ledit interstice, ladite au moins une sortie étant située à une extrémité opposée dudit interstice ; et

c) un contrôleur configuré et/ou programmé pour contrôler le fonctionnement du dispositif de séparation.

12. Système de la revendication 11, dans lequel ledit contrôleur est configuré et/ou programmé pour déterminer le % de volume tassé total dans ladite suspension.

13. Système de la revendication 12, dans lequel ledit contrôleur est configuré et/ou programmé pour déterminer un volume d'une suspension de cellules biologiques et de microsupports devant être introduite dans ledit dispositif de séparation.

14. Système de l'une quelconque des revendications 11 à 13, dans lequel ladite membrane poreuse a une taille de pores d'environ 30 $\mu$m.

Fig. 1

Fig. 2

*Fig. 3*

*Fig. 4*

**FIG. 5a**

**FIG. 5b**

EP 3 199 617 B1

FIG. 6

**EP 3 199 617 B1**

**REFERENCES CITED IN THE DESCRIPTION**

**Patent documents cited in the description**

- US 2014199680 A
- US 5194145 A, Schoendorfer
- US 2012028492 W
- US 61537856
- US 2012028522 W
- US 2012054859 W
- US 57453914
- US 5053121 A
- US 61451903
- US 122855